# EUROPEAN PATENT APPLICATION

(11) **EP 4 190 348 A1**
(43) Date of publication of application: **07.06.2023**
(21) Application number: 21759232.8
(22) Date of filing: 19.07.2021
(51) Int. Cl.: A61K 39/00, A61P 11/00

(54) **USE OF EPIDERMAL GROWTH FACTOR DEPLETING AGENTS IN THE TREATMENT OF THE CHRONIC OBSTRUCTIVE PULMONARY DISEASE**

(30) Priority: 30.07.2020 CU 20200034
(71) Applicant: Centro de Inmunologia Molecular, Playa, La Habana 11300 (CU)
(72) Inventor: MACÍAS ABRAHAM, Amparo Emilia, La Habana 10400 (CU); CROMBET RAMOS, Tania, La Habana 10800 (CU); LEÓN MONZÓN, Kalet, La Habana 17100 (CU); SAAVEDRA HERNÁNDEZ, Danay, La Habana 10800 (CU); SANTOS MORALES, Orestes, La Habana 17100 (CU); NENINGER VINAGERAS, Elia, La Habana 10200 (CU); PINO ALFONSO, Pedro Pablo, La Habana 10200 (CU); HERNANDEZ REYES, Jenysbel de la Caridad, La Habana 17100 (CU); REID, Mary, Buffalo, New York 14263 (US); LEE, Kelvin, Buffalo, New York 14263 (US)
(74) Representative: Capitán García, Maria Nuria
(86) International application number: PCT/CU2021/050006
(87) International publication number: WO 2022/022756

(57) **Abstract**

The present invention is related to the fields of Biotechnology and Medicine. Particularly, it describes the use of epidermal growth factor (EGF) deprivation agents that contribute to lowering and/or depleting serum epidermal growth factor levels, which has implications in the treatment of the chronic obstructive pulmonary disease. These agents can be vaccine compositions comprising as active principle the conjugate between recombinant human EGF and a carrier protein.

## Description

### SCOPE OF THE TECHNIQUE

The present invention relates to the fields of Biotechnology and Medicine. It is particularly related to the use of epidermal growth factor (EGF) deprivation agents, specifically vaccine compositions comprising Epidermal Growth Factor (EGF) as active principle and a carrier protein in the chronic obstructive pulmonary disease (COPD) treatment

### BACKGROUND

The COPD is characterized by the presence of chronic and poorly reversible airflow obstruction associated with an abnormal and chronic inflammatory reaction that leads to airway, parenchyma and pulmonary arteries remodelling, it also has systemic effects (Fletcher C. et al. (1977) Br Med J. 1 (6077): 1645-1648). The prevalence of and mortality by COPD have significantly increased worldwide, this disease is currently the fourth global cause of mortality (NIH Publication No 2701A, March 2001 available at: www.goldcopd.com). Continued exposure to tobacco smoke and biomass combustion products in closed environments has been associated with COPD (Pérez-Padilla R. et al. (1996) Am J Respir Crit Care Med. 154: 701-706; Orozco-Levi M. et al. Eur Respir J. (2006); 27 (3): 542-546). Homozygous alpha-1 antitrypsin deficiency is associated with early emphysema in smokers, leading to the onset of COPD (Alpha-1-Antitrypsin Deficiency Registry Study Group. (1998) Am J Respir Crit Care Med. 158 (1): 49 -59). COPD is the greatest risk factor for lung cancer in smokers, a population in which the risk of suffering from this type of cancer is 4.5 times higher. Also, the disease is found in 50-90% of lung cancer patients. Lung cancer is the leading cause of morbidity and mortality in COPD patients as well (Anthonisen N.R. et al. (2005) Ann Intern Med.; 142: 233-239).

The involvement of the EGF and its receptor (EGFR) in the pathogenesis of COPD has been described. It has been shown that the positive regulation of the EGFR achieved through the addition of its EGF and TGFα ligands in the epithelial cells of the respiratory tract, induces the expression of the MUC5AC and MUC2 genes, which are responsible for the production of mucus in the respiratory tract. The expression of most receptors and ligands of the EGF/EGFR system is higher in damaged epithelium than in intact epithelium. Overexpression of the EGF/EGFR and/or other ligands of the family can lead to changes in normal airway epithelium such as metaplasia or epithelial hyperplasia (Willem I. et al. Am J Clin Pathol. (2006) 125: 184 -192). Human eosinophils induce mucin production by airway epithelial cells through EGFR activation (Burgel P. R. et al. (2001) J Immunol. 167: 5948-5954).

Currently the treatment of COPD is fundamentally palliative, that is, directed mainly towards the control of the clinical symptoms of the disease. In clinical practice, the use of drugs aimed at the etiopathogenesis of the disease is under clinical investigation. The use of the inhibitor of tyrosine kinase activity called BIBW 2948, was studied in COPD, but the treatment regimens employed not only did not decrease mucus production in patients but also resulted in the appearance of serious adverse effects, such as increased transaminase enzymes. Although higher *in vivo* doses demonstrated an effect on mucus production reduction, the adverse effects reported in the clinical trial did permit dose escalation (Woodruff W. et al. (2010) Am J Respir Crit Care Med 181: 438- 445).

US 2011/0192396 patent application describes the use of pharmaceutical compositions containing the EGF and a buffered or saline solution as a suitable pharmaceutical vehicle for the treatment of COPD. The effect of the EGF on mucus hypersecretion, protection of alveolar cells and improvement of pathological tissue manifestations in pulmonary emphysema models has been demonstrated in *in vitro* and *in vivo* preclinical studies.

It is appropriate to highlight that no technical solution or scientific publication before the present invention describes the use of immunotherapeutic agents that cause the deprivation of the EGF in sera by active immunization has been described. The novelty of the present invention lies in providing a treatment for COPD with less toxic, EGF blocking therapeutic alternatives, which consist in the use of vaccine compositions that induce the production of specific antibodies against the EGF. The safety demonstrated by these vaccine compositions in cancer treatments allows for their use in prolonged therapies, which results in an increase in the efficacy of these treatments. In the present invention, its inventors achieved the improvement of the clinical symptoms of COPD by administering the aforementioned vaccine compositions to patients suffering from this disease.

### BRIEF DESCRIPTION OF THE INVENTION

In one embodiment, the object of the present invention is the use of EGF-depleting immunotherapeutic agents for the treatment of the chronic obstructive pulmonary disease. In particular, said immunotherapeutic agents refer to vaccine compositions that induce the production of specific antibodies against the EGF. These vaccine compositions comprise as active principle a conjugate between the recombinant human EGF and a carrier protein that can be: cholera toxin B subunit, tetanus toxoid, KLH and P64k from *Neisseria meningitidis.* Additionally, the vaccine composition has an adjuvant that is selected from the group comprising: incomplete Freund's adjuvant, squalene based adjuvants, synthetic origin adjuvants, mineral origin adjuvants, plant origin adjuvants, animal origin adjuvants, particulate proteic adjuvants and liposomes.

In another embodiment, the present invention provides a method of treatment for a subject in need thereof comprising the administration of the aforementioned vaccine compositions by intramuscular route preferably at a dose range between 20-70 µL/kg, with the first four doses being administered every 14 days and the remaining ones every 28 days for a minimum period of 6 months.

### DETAILED DESCRIPTION OF THE INVENTION

### Vaccine compositions

The vaccine compositions provided in the present invention, which are used as EGF deprivation agents in the treatment of COPD, are all those that induce the production of antibodies against the EGF that contribute to decreasing and/or depleting serum EGF levels.

Examples of said vaccine compositions are all those that comprise as an active principle the conjugate between recombinant human EGF (rhEGF) and a carrier protein. Said carrier protein is selected from the group, without being limited to it, comprising: cholera toxin B subunit, tetanus toxoid, KLH and P64k from *Neisseria meningitidis.* Additionally, the aforementioned vaccine compositions comprise an adjuvant that is selected from the group comprising: incomplete Freund's adjuvant, squalene based adjuvants, synthetic origin adjuvants, mineral origin adjuvants, plant origin adjuvants, animal origin adjuvants, particulate proteic adjuvants and liposomes.

### Treatment methods

Another embodiment of the present invention, provides a method of treatment with the above-described vaccine compositions. Patients diagnosed with COPD, following the GOLD criteria (Global Initiative Guidelines for Chronic Obstructive Pulmonary Disease) (NHLBI / WHO workshop report. NIH Publication No 2701A, March 2001, available at: www goldcopd com), are eligible to receive this treatment.

According to these criteria, COPD is characterized by the presence of chronic and poorly reversible airflow obstruction associated with an abnormal inflammatory reaction. Its main clinical symptoms are: cough, sputum production and dyspnea or feeling suffocated when walking or increasing the level of an activity, which increasingly worsens over the years. Airflow obstruction is diagnosed by spirometry when the ratio of: Forced expiratory volume in the first second (FEV1) / Forced vital capacity (FVC) after post-bronchodilator test is less than 0.7 (or below the lower limit of normal in subjects older than 60 years). In the present invention, the COPD patients immunized with the vaccine compositions described in the present invention will show clinical benefit. The term clinical benefit in the context of the present invention refers to the fact that treatment with such agents improves and/or stabilizes at least one of the respiratory clinical symptoms patients present.

In the context of this invention, the term therapeutic effect refers to the beneficial or desirable impact of a treatment in general, for example, the improvement or remission of the symptoms of the disease.

The administration of the vaccine compositions that comprise the EGF as active principle is preferably performed by intramuscular route, the first 4 doses being administered every 14 days and the remaining ones every 28 days, with an allowable delay time range of 3 days. The dose range at which said compositions will be administered will be between 20-70 µL /kg of body weight or 20-70 µg of total proteins per kilogram of body weight or up to 5 mg of total proteins, more preferably 30-60 µg/kg . The treatment will have a minimum duration of 6 months. This treatment schedule can change in frequency and dosage according to the results obtained and can be optimized taking into account the antibody titers generated and the improvement and/or stabilization of clinical symptoms.

The present invention is further elaborated with the following examples and figures. However, these examples should not be construed as limiting the scope of the invention.

### BRIEF DESCRIPTION OF THE FIGURES

**FIGURE 1****.** Baseline serum EGF levels in patients with grade III/IV COPD (severe and very severe stages, according to GOLD criteria), compared with untreated and treated with chemotherapy stage III/IV non-small cell lung cancer (NSCLC) patients.
**FIGURE 2****.** Immunodeprivation of serum EGF levels and anti-EGF antibody titers achieved over time in a grade III COPD patient treated with CIMAvax-EGF.
**FIGURE 3****.** Percentage of inhibition of phosphorylation by anti-EGF antibodies.

### EXAMPLES

### Example 1. Both COPD patients and NSCLC patients have high baseline serum EGF levels.

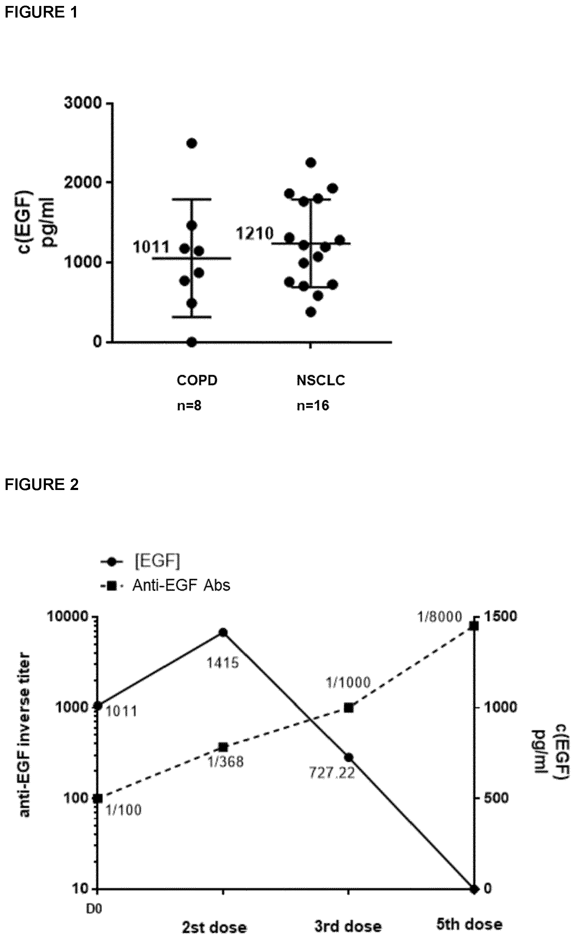

The baseline serum EGF levels of a sample of patients with grade III/ IV COPD and a group of NSCLC patients were determined by ELISA (González, EC et al. J Immunoassay Immunochem. (2016) 16: 1-12). Similar levels of serum EGF were found in COPD and NSCLC patients (Figure 1).

### Example 2. Treatment with CIMAvax-EGF induces anti-EGF antibodies while immunodeprivation serum EGF and improving the clinical symptoms of COPD.

A patient with severe COPD (grade III according to the GOLD criteria) was immunized with 2.4 mg of the active ingredient of the therapeutic vaccine CIMAvax-EGF, receiving a total of five doses intramuscularly. The anti-EGF antibody titers were quantified by ELISA (Gonzalez G. A. et al. Ann Oncol (1998); 9: 431-435). Serum EGF levels during treatment were determined by ELISA (González, EC et al. J Immunoassay Immunochem. (2016) 16: 1-12).

Figure 2 shows that immunodeprivation of serum EGF levels related to an increase in anti-EGF antibody titers in the patient was achieved during treatment.

Additionally, spirometry was performed before starting and after finishing the treatment with the vaccine. Table 1 shows the results of FEV1 the best indicator of the severity of airflow obstruction.

**Table 1. FEV1 behavior of a patient with grade III COPD treated with CIMAvax-EGF.**

| **Treatment with CIMAvax-EGF** | **FEV1 %** | **GOLD criteria** |
|---|---|---|
| Before | 48 | Grade III severe greater than or equal to 30% and less than 50% |
| After | 60 | Grade II moderate greater than or equal to 50% and less than 80% |

As can be seen in the results displayed in Table 1, treatment with CIMAvax-EGF improved the clinical respiratory symptoms in the treated patient, decreasing the degree of severity according to GOLD criteria.

### Example 3. Treatment with CIMAvax-EGF induces anti-EGF antibodies with the ability to inhibit EGF receptor phosphorylation.

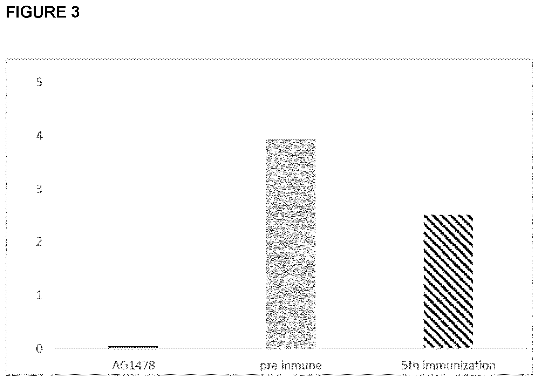

A patient with severe COPD (grade III according to GOLD classification) was immunized with 2.4 mg of the active ingredient of the therapeutic vaccine CIMAvax-EGF, receiving a total of 8 doses intramuscularly. The inhibition capacity of the serum obtained previously to the first immunization and on the fifth immunization was assessed by means of the Western Blot technique, incubating them for one hour with tumor cells expressing the EGF receptor. The AG1478 antibody was used as inhibition control. Figure 3 shows that the antibodies generated with the vaccination inhibit the EGF receptor phosphorylation in the presence of EGF (36% inhibition).

## Claims

1. Use of an epidermal growth factor (EGF)-depleting immunotherapeutic agent in the treatment of the chronic obstructive pulmonary disease.

2. Use according to claim 1 wherein the immunotherapeutic agent is a vaccine composition that induces the production of specific antibodies against the EGF.

3. Use according to claim 2, wherein the vaccine composition comprises as an active principle the conjugate between the recombinant human EGF and a carrier protein.

4. Use according to claim 3 wherein the carrier protein is selected from the group comprising:
- cholera toxin B subunit
- tetanus toxoid,
- KLH and
- P64k from *Neisseria meningitidis.*

5. Use according to claim 3 wherein the vaccine composition additionally has an adjuvant that is selected from the group comprising:
- incomplete Freund's adjuvant,
- squalene based adjuvants,
- adjuvants of synthetic origin,
- adjuvants of mineral origin,
- adjuvants of plant origin,
- adjuvants of animal origin,
- particulate proteic adjuvants and
- liposomes.

6. A method for treating a subject in need thereof comprising the administration of the vaccine compositions of any of claims 2-5 by intramuscular route at a dose range betwen 20-70 µL /kg, with the first four doses being administered every 14 days and the remaining ones every 28 days for a minimum period of 6 months.
